# EUROPÄISCHE PATENTSCHRIFT

(11) **EP 3 960 141 B1**
(45) Veröffentlichungstag und Bekanntmachung des Hinweises auf die Patenterteilung: **14.02.2024**
(21) Anmeldenummer: 20193645.7
(22) Anmeldetag: 31.08.2020
(51) Int. Cl.: A61F 13/496, A61F 13/49

(54) **HYGIENEARTIKEL IN HÖSCHENFORM**
HYGIENE ARTICLE IN PANTY FORMAT
ARTICLE D'HYGIÈNE SOUS LA FORME DE CULOTTE

(43) Veröffentlichungstag der Anmeldung: 02.03.2022
(73) Patentinhaber: W. Pelz GmbH & Co. KG, 23812 Wahlstedt/Holstein (DE)
(72) Erfinder: BASTIAN, Nikolas, 22605 Hamburg (DE)
(74) Vertreter: Hauck Patentanwaltspartnerschaft mbB

(56) Entgegenhaltungen:
- EP-A1- 1 787 610
- EP-A1- 3 117 809
- WO-A1-2014/208635
- WO-A1-2018/209629
- WO-A1-2019/193906
- DE-A1-102010 019 702
- US-A1- 2018 140 477

## Beschreibung

Die Erfindung betrifft einen Hygieneartikel in Höschenform.

Hygieneartikel in Höschenform (Pants) dienen dem Auffangen von Ausscheidungen des menschlichen Körpers, wie Harn oder Stuhl. Sie werden insbesondere von Personen benutzt, die unter Inkontinenz leiden, vorwiegend von Senioren. Derartige Pants werden aus Fasermaterialien, insbesondere aus zellulosehaltigen und/oder Superabsorber enthaltenden Fasermaterialien, sowie aus Kunststofffolien und Vliesstoffen hergestellt. Es handelt sich hierbei um Einmalartikel (Disposables), die nach einmaligem Gebrauch weggeworfen werden. Die Hersteller sind bestrebt, die Eigenschaften von Pants denen von herkömmlicher Unterwäsche anzunähern, um Tragkomfort und Akzeptanz beim Verbraucher zu verbessern.

Grundsätzlich weisen Hygieneartikel in Höschenform ein Vorderteil, ein Rückenteil sowie ein Vorderteil und Rückenteil miteinander verbindendes Einsatzteil auf, wobei Vorderteil und Rückenteil am oberen Rand eine Hüftöffnung umgrenzen, Vorderteil, Rückenteil und Einsatzteil an zwei seitlichen Rändern Beinöffnungen umgrenzen, das Einsatzteil im Schrittbereich einen Saugkern aufweist und Vorderteil und Rückenteil parallel zu den oberen Rändern verlaufende und im entspannten Zustand Vorderteil und Rückenteil raffende elastische Fäden aufweisen.

Die Elastifizierungsmittel sind elastische Fäden (z. B. Elasthan-Fäden), die auf die Teile des Hygieneartikels im gespannten Zustand aufgebracht sind, sodass sie diesen im entspannten Zustand raffen. Die parallel zum oberen Rand verlaufenden Elastifizierungsmittel dienen dazu, den Hygieneartikel im Bereich des Unterleibes und der Hüfte zu fixieren. Zur Fixierung im Hüftbereich kann der Hygieneartikel einen elastischen Bund aufweisen. Vorzugsweise sind zusätzlich an den Beinöffnungen Elastifizierungsmittel vorhanden, um den Hygieneartikel eng an die Beine des Trägers anzulegen.

Herkömmliche Pants weisen im Bereich von Vorderteil und Rückenteil (zusammenfassend als "Chassis" bezeichnet) wenige, weit auseinanderliegende elastische Fäden auf. Beispielsweise beträgt der Abstand von Faden zu Faden etwa 8 mm und die Faserstärke 540 dtex. Die elastischen Fäden schneiden in die Haut ein, machen das Chassis sperrig und kratzig und erzeugen auf der Haut ein Fremdkörpergefühl, dass vom Träger als störend empfunden wird. Zur sicheren Fixierung des Hygieneartikels am Körper des Trägers sind die Elastifizierungsmittel im Bereich des Bundes stark ausgeprägt, sodass die Hygieneartikel im entspannten Zustand insbesondere an der Hüftöffnung entsprechend stärker gerafft sind und mit der entsprechend hoher Spannung am Hüftbereich anliegt.

Die EP 3 117 809 A beschreibt einen Hygieneartikel, der ein Vorderteil, ein Rückenteil und ein diese miteinander verbindendes Einsatzteil aufweist. Das Einsatzteil ist an den Enden durch zurückgefaltete Abschnitte des äußeren Flachmaterials vom Vorderteil und Rückenteil abgedeckt. Die oberen Ränder des äußeren Flachmaterials von Vorderteil und Rückenteil und die schmalen Ränder des Einsatzteiles überlappen einander. Hierfür erstreckt sich das Einsatzteil fast bis zu den oberen Rändern von Vorderteil und Rückenteil. Durch die zurückgeschlagenen Abschnitte der äußeren Flachmaterialien von Vorderteil und Rückenteil und das beinahe bis zu den oberen Rändern derselben sich erstreckende Einsatzteil sind über große Flächenbereiche mehrere Materiallagen übereinander angeordnet. Neben den einander überlappenden Bereichen weist der Hygieneartikel Bereiche mit weniger Materiallagen und geringerer Wandstärke auf. Infolgedessen trägt der Hygieneartikel stark auf und kann sich im Unterschied zu normaler Unterwäsche deutlich abzeichnen.

Davon ausgehend liegt der Erfindung die Aufgabe zugrunde, einen Hygieneartikel in Höschenform mit verbessertem Tragekomfort zu schaffen.

Die Aufgabe wird durch einen Hygieneartikel in Höschenform mit den Merkmalen von Anspruch 1 gelöst. Vorteilhafte Ausführungsarten des Hygieneartikels sind in Unteransprüchen angegeben.

Der erfindungsgemäße Hygieneartikel in Höschenform weist ein Vorderteil und ein Rückenteil sowie einen Vorderteil und Rückenteil miteinander verbindendes Einsatzteil (Insert) auf, wobei Vorderteil und Rückenteil am oberen Rand eine Hüftöffnung umgrenzen, Vorderteil, Rückenteil und Einsatzteil an zwei seitlichen Rändern Beinöffnungen umgrenzen, das Einsatzteil im Schrittbereich einen Saugkern aufweist und Vorderteil und Rückenteil parallel zueinander verlaufende und im entspannten Zustand Vorderteil und Rückenteil raffende elastische Fäden aufweist und die elastischen Fäden in senkrecht zum oberen Rand verlaufender Richtung im Mittel eine Dichte von mindestens zwei Fäden pro Zentimeter, vorzugsweise mindestens 2,5 Fäden pro Zentimeter, vorzugsweise mindestens vier Fäden pro Zentimeter aufweisen, dadurch gekennzeichnet, dass das Einsatzteil an den schmalen Seiten durch Streifen aus einem Vliesmaterial abgedeckt ist.

Dem erfindungsgemäßen Hygieneartikel liegt die Erkenntnis zugrunde, dass ein direkter Zusammenhang zwischen der Weichheit, der Flexibilität, Formstabilität und einem angenehmen Tragegefühl des Hygieneartikels auf der nackten Haut des Trägers und der Dichte der elastischen Fäden in Vorderteil und Rückenteil besteht. Als Maß für die Dichte wird die Anzahl der elastischen Fäden pro Längeneinheit (Zentimeter) in einer senkrecht zum oberen Rand verlaufenden Richtung herangezogen. Es wurde gefunden, dass bei einem Hygieneartikel, der im Mittel eine Dichte von mindestens zwei Fäden pro Zentimeter, vorzugsweise mindestens 2,5 Fäden pro Zentimeter, vorzugsweise mindestens vier Fäden pro Zentimeter aufweist, sich ein wesentlich verbesserter Tragekomfort einstellt. Dabei wird als Mittel der Dichte die Anzahl der Fäden pro Zentimeter verstanden, die sich aus dem Quotienten der gesamten Anzahl der zum oberen Rand parallelen elastischen Fäden des Vorderteils/Rückenteils (d.h. des Vorderteils und/oder des Rückenteils) und der maximalen Höhe des Hauptteils des Vorderteils/Rückenteils ergibt. Der Hauptteil des Vorderteils/Rückenteils ist derjenige Teil des Vorderteils/Rückenteils, der keinen elastischen Bund (Gürtel) aufweist, in dem elastische Fäden mit einer höheren Dichte angeordnet sind. Bei einem Vorderteil/Rückenteil mit Gürtel ist der Hauptteil also der unter dem Gürtel angeordnete Teil des Vorderteils/Rückenteils und bei einem Vorderteil/Rückenteil ohne Gürtel ist der Hauptteil das gesamte Vorderteil/Rückenteil.

Aufgrund der hohen Dichte der elastischen Fäden bildet das Chassis im entspannten Zustand weniger Rüschen, legt sich glatter an den Körper des Trägers an und hält sicherer die angelegte Position ein. Insbesondere kann sich der Hygieneartikel eng und ohne Bildung von Materialanhäufungen an Knochen im Beckenbereich anschmiegen. Hierdurch ist es auch möglich, die Fäden mit einer geringeren Vorspannung als bei herkömmlichen Pants auf Vorderteil und Rückenteil aufzubringen, ohne dass der Hygieneartikel rutscht. Da der Hygieneartikel glatt und ohne Einzuschneiden an der Haut anliegt, sicher sitzt, sich an die Körperkontur anschmiegt, nicht aufträgt und sich unter der Kleidung nicht abzeichnet, wird das Tragen des Hygieneartikels als besonders angenehm empfunden.

Dadurch, dass das Einsatzteil an den schmalen Seiten durch Streifen aus einem Vliesmaterial abgedeckt ist, wird ein besonders hoher Tragekomfort erreicht, da die schmalen Seiten des Einsatzteils direkt an der Haut des Trägers anliegen.

Gemäß der Erfindung verlaufen die elastischen Fäden entlang zum oberen Rand von Vorderteil/Rückenteil parallelen Achsen. Dies ist besonders vorteilhaft für die Herstellung der Hygieneartikel in einem Hochgeschwindigkeitsprozess. Gemäß der Erfindung verlaufen die elastischen Fäden geradlinig oder wellenförmig (z.B. sinus-, rechteck-, dreieck- oder sägezahnförmig) entlang der Achsen. Der geradlinige Verlauf ist besonders vorteilhaft für die Herstellung. Mit einem wellenförmigen Verlauf wird eine Elastizität des Hygieneartikels in einer senkrecht zu den parallelen Achsen verlaufenden Richtung erreicht.

Gemäß der Erfindung sind die elastischen Fäden in einer senkrecht zum oberen Rand verlaufenden Richtung zumindest in einem Abschnitt des Vorderteils und des Rückenteils gleichmäßig über das Vorderteil und das Rückenteil verteilt. Hierdurch wird ein Einschneiden von einzelnen Fäden in die Haut des Trägers besonders wirksam vermieden und ein besonders gleichmäßiger, angenehmer, sicherer und anpassungsfähiger Sitz des Hygieneartikels am Körper des Trägers erreicht. Unter einer gleichmäßigen Verteilung der elastischen Fäden über das Vorderteil und das Rückenteil wird verstanden, dass die Fäden in einer zum oberen Rand senkrechten Richtung den gleichen Abstand voneinander haben. Gemäß einer weiteren Ausführungsart sind die Fäden über die gesamte Höhe des Vorderteils und des Rückenteils gleichmäßig verteilt. Gemäß einer weiteren Ausführungsart sind die Fäden über das Hauptteil des Vorderteils/Rückenteils gleichmäßig verteilt. Gemäß einer weiteren Ausführungsart sind die Fäden über den elastischen Bund gleichmäßig verteilt. Gemäß einer weiteren Ausführungsart, bei dem der Hygieneartikel am oberen Rand einen umlaufenden elastischen Bund aufweist, sind die elastischen Fäden unterhalb des Bundes gleichmäßig über das Vorderteil und das Rückenteil verteilt. Gemäß einer weiteren Ausführungsart, bei dem auch der Bund elastische Fäden aufweist, sind die elastischen Fäden über die gesamte Höhe des Vorderteils und des Rückenteils gleichmäßig verteilt oder im Bereich des Bundes anders verteilt als darunter.

Gemäß einer weiteren Ausführungsart haben die Fäden im Hauptteil des Vorderteils/Rückenteils im Mittel eine andere, vorzugsweise geringere, Dichte als im Bund. Gemäß einer weiteren Ausführungsart beträgt die mittlere Dichte im Hauptteil 2,5 Fäden/cm und im Bund 4 Fäden/cm.

Gemäß einer weiteren Ausführungsart weisen die elastischen Fäden in einer senkrecht zum oberen Rand verlaufenden Richtung zumindest über einen Abschnitt des Vorderteils und des Rückenteils denselben Titer auf. In der Textiltechnik ist der Titer ein gebräuchliches Maß für die Feinheit von Fäden. Dadurch, dass die elastischen Fäden von Vorderteil und Rückenteil denselben Titer aufweisen, wird ein besonders glatt anliegender, sicher sitzender und nicht auftragender Hygieneartikel erreicht. Gemäß der Erfindung weisen Vorderteil und Rückenteil über die gesamte Höhe elastische Fäden mit demselben Titer auf. Gemäß einer anderen Ausführungsart weißt der Hygieneartikel am oberen Rand einen umlaufenden elastischen Bund auf und weisen die elastischen Fäden im Hauptteil vom Vorderteil/Rückenteil unterhalb des Bundes denselben Titer auf. Gemäß einer weiteren Ausführungsart weist der elastische Bund ebenfalls elastische Fäden auf und weisen die elastischen Fäden im Bereich des Bundes denselben Titer oder einen anderen Titer, vorzugsweise größeren Titer, als die Fäden unterhalb des elastischen Bundes, auf.

Gemäß einer weiteren Ausführungsart weisen die elastischen Fäden einen Titer von 400 bis 750 dtex auf. Gemäß einer weiteren Ausführungsart weisen die elastischen Fäden im Bereich des Hauptteils von Vorderteil/Rückenteil einen Titer von 400 bis 600 dtex, vorzugweise 450 bis 500 dtex, vorzugsweise von 470 dtex auf, und im Bereich des Bundes von 600 bis 750 dtex, vorzugsweise von 680 dtex.

Gemäß der Erfindung weisen das Vorderteil und/oder das Rückenteil ein Laminat aus zwei Lagen Vliesstoff mit zwischen den beiden Lagen angeordneten und an mindestens einer Lage befestigten elastischen Fäden auf oder sind durch ein solches Laminat gebildet. Durch die Ausbildung von Vorderteil und/oder Rückenteil als Laminat aus Vliesstoff und elastischen Fäden wird eine besonders dünne und hautfreundliche Ausführung und ein besonders hoher Tragekomfort erreicht.

Die nachfolgenden Ausführungsarten sind insbesondere vorteilhaft für eine kostengünstige Herstellung mit hoher Geschwindigkeit: der Erfindung sind die elastischen Fäden an mindestens einer Lage aus Vliesstoff angeklebt und/oder durch Ultraschallbschweißen an mindestens einer Lage aus Vliesstoff befestigt. Gemäß der Erfindung sind die elastischen Fäden im gedehnten Zustand auf eine Lage aus einem flach ausgebreiteten Vliesstoff aufgebracht und an dieser befestigt. Gemäß einer weiteren Ausführungsart sind die elastischen Fäden in einer senkrecht zum oberen Rand verlaufenden Richtung überall mit derselben Vorspannung auf eine Lage aus einem Vliesstoff aufgebracht und an dieser befestigt.

Gemäß der Erfindung weisen das Vorderteil und das Rückenteil am oberen Rand einen elastischen Bund auf. Gemäß der Erfindung haben das Vorderteil und das Rückenteil innerhalb des elastischen Bundes parallel zum oberen Rand verlaufende und im entspannten Zustand den Bund raffende elastische Fäden. Durch den elastischen Bund wird der Hygieneartikel noch mehr an die Erscheinungsform herkömmlicher Unterwäsche angenähert. Ferner kann durch den elastischen Bund ein noch sicherer Sitz des Hygieneartikels am Körper des Trägers erreicht werden. Zudem kann der Hygieneartikel im Bereich des elastischen Bundes besonders reißfest ausgebildet werden, sodass der Hygieneartikel beim Anlegen nicht reißt. Der elastische Bund kann von den tiefer angeordneten Abschnitten (d.h. den unterhalb des Bandes angeordneten Abschnitten) von Vorderteil und Rückenteil durch eines oder mehrere der folgenden Merkmale abgegrenzt sein:
(i) zwischen den tiefer angeordneten Abschnitten von Vorderteil und Rückenteil und dem elastischen Bund ist eine von elastischen Fäden freie Zone vorhanden,
(ii) in dem elastischen Bund ist die Dichte der Fäden größer als in den tiefer angeordneten Abschnitten von Vorderteil und Rückenteil,
(iii) in dem elastischen Bund weisen die elastischen Fäden einen höheren Titer auf als in den tiefer angeordneten Abschnitten von Vorderteil und Rückenteil,
(iv) in dem elastischen Bund sind die elastischen Fäden mit einer größeren Vorspannung aufgebracht als in den tiefer angeordneten Abschnitten von Vorderteil und Rückenteil.

Gemäß der Erfindung ist das Vorderteil rechteckig und/oder ist das Rückenteil in einem oberen Abschnitt rechteckig und in einem unteren Abschnitt trapezförmig. Dies ist vorteilhaft für eine Passform, die sich eng an den Körper des Trägers anschmiegt, sicher sitzt, wenig aufträgt, Bein- und Bewegungsfreiheit begünstigt und mit relativ geringem Materialaufwand hergestellt werden kann.

Der Hygieneartikel kann insbesondere eine Passform aufweisen, die der Passform von Unterwäsche entspricht, die als Hipster (Hüfthose) bezeichnet wird.

Gemäß der Erfindung sind das Vorderteil und das Rückenteil in Seitennähten an den seitlichen Rändern durch Heißkleber und/oder Prägemuster miteinander verbunden, wobei vorzugsweise in den Seitennähten zwischen den einander überlappenden seitlichen Rändern von Vorderteil und Rückenteil Streifen aus einem Vliesmaterial eingelegt sind, die von den inneren Rändern der Seitennähte vorstehen und die angrenzenden Randbereiche des Vorderteils und des Rückenteils überdecken. Durch die Streifen aus Vliesmaterial wird der Tragekomfort weiter verbessert, da diese einen direkten Kontakt der Seitennähte mit der Haut des Trägers verhindern, der als unangenehm empfunden wird.

Gemäß der Erfindung überdecken die Endbereiche des Einsatzteils untere Randbereiche des Vorderteils und des Rückenteils und sind an diesen befestigt und sind die elastischen Fäden im Bereich des Einsatzteils durchtrennt. Hierdurch wird verhindert, dass beim Anlegen des Hygieneartikels die Dehnung der elastischen Fäden auf das Einsatzteil und den Saugkern einwirkt und diese zusammenziehen oder anderweitig verformen. Hierdurch wird der Tragkomfort im Schrittbereich verbessert. Zu diesem Zweck sind gemäß der Erfindung die elastischen Fäden im Bereich des Einsatzteils nicht an den Lagen aus Vliesstoff befestigt.

Die elastischen Fäden können jeweils aus einer Vielzahl von Fasern zusammengesetzt oder Einzelfäden (Endlosfasern) sein. Gemäß der Erfindung sind die elastischen Fäden Elasthanfäden. Elasthanfäden sind aus Fasern aus Elasthan gebildete Fäden. Gemäß einer weiteren Ausführungsart bestehen die elastischen Fäden im Wesentlichen oder vollständig aus Polyurethan (z.B. aus Lycra^{®}). Gemäß einer anderen Ausführungsart sind die elastischen Fäden Gummifäden.

Gemäß einer weiteren Ausführungsart umfasst der Hygieneartikel mindestens eines der folgenden Merkmale:
- Das Einsatzteil hat eine Breite von max. 110 mm bis 90 mm, vorzugsweise von 100 mm. Dabei wird ein besonders guter Tragekomfort im Schrittbereich ohne Einschränkung der Flüssigkeitsaufnahme erreicht.
- Der Saugkern umfasst mindestens eine Lage aus einem Gemisch aus Cellulosefasern und Superabsorber. Hierdurch wird eine besonders hohe Aufnahmekapazität von Körperflüssigkeit erreicht. Gemäß einer weiteren Ausführungsart umfasst der Saugkern eine mittlere Lage aus einem Gemisch aus Cellulosefasern und Superabsorbern und zwei äußeren Lagern aus Cellulosefasern.
- Der Saugkern hat in der Mitte seine größte Dicke und eine zu den Enden hin sich verringernde Dicke. Hierdurch wird eine besonders hohe Flüssigkeitsaufnahmekapazität im Bereich der Körperöffnungen erreicht und zugleich das Auftragen des Hygieneartikels im Bereich von Scham und Po verringert.
- Die Dicke des Saugkerns verringert sich zu den Enden des Kerns hin in den die unteren Randbereiche von Vorderteil und Rückenteil überdeckenden Endbereichen des Einsatzteils. Hierdurch wird das Auftragen im Bereich von Scham und Po weiter herabgesetzt.
- Der Saugkern ist in ein Nonwoven eingeschlagen, das den Saugkern C-förmig umhüllt. Hierdurch kann das Fasermaterial des Kerns bei der Produktion und beim Tragen zusammengehalten werden.
- Auf der Innenseite des Saugkerns ist eine Akquisitionsschicht aufgebracht. Hierdurch kann eine gleichmäßige Verteilung von Körperflüssigkeit Ausnutzung der Aufnahmekapazität des Kerns erreicht werden.
- Der Saugkern und die Akquisitionsschicht sind von einem weiteren Nonwoven als Topsheet umschlossen. Hierdurch kann das Einsatzteil zusammengehalten und der Tragekomfort im Schrittbereich verbessert werden.
- An den seitlichen Randbereichen des Einsatzteils sind elastische Fäden aufgebracht. Hierdurch kann eine enge Anlage des Einsatzteils an dem Schrittbereich erreicht werden. Gemäß einer bevorzugten Ausführungsart bestehen die elastischen Fäden des Einsatzteils aus denselben Materialien wie die elastischen Fäden des Vorderteils und des Rückenteils.
- An den beiden Längsseiten des Saugkerns sind auf der Innenseite des Einsatzteils Abdichtstreifen (Cuffs) angeordnet, die an den äußeren Rändern mit elastischen Fäden verbunden sind. Hierdurch kann eine besonders gut abdichtende Anlage des Einsatzteils am Schrittbereich erreicht werden. Gemäß einer weiteren Ausführungsart handelt es sich bei den restlichen Fäden um dieselben Materialien wie bei den elastischen Fäden des Vorderteils und des Rückenteils.
- Die Abdichtstreifen haben eine Breite 25 bis 18 mm, vorzugsweise von 22 mm. Hierdurch wird eine besonders gute Abdichtung bei hohem Tragekomfort erreicht.

Die Erfindung wird nachfolgend anhand der anliegenden Zeichnungen eines Ausführungsbeispiels näher erläutert. In den Zeichnungen zeigen:
- Fig. 1: einen Hygieneartikel in Höschenform flach ausgebreitet in einer Draufsicht auf die Innenseite;
- Fig. 2: derselbe Hygieneartikel in einem Längsschnitt;
- Fig. 3: derselbe Hygieneartikel in einem Querschnitt
- Fig. 4: Saugkern desselben Hygieneartikels in einem Längsschnitt;
- Fig. 5: Seitennaht desselben Hygieneartikels in einer Perspektivansicht schräg von der Seite;
- Fig. 6: Seitennaht desselben Hygieneartikels in einem Querschnitt.

In der vorliegenden Anmeldung beziehen sich die Angaben "oben" und "unten", davon abgeleitete Angaben wie "darunter" und "darüber" sowie "Vorderteil" und "Rückenteil" auf die Gebrauchsstellung des Hygieneartikels am Körper eines aufrechtstehenden Benutzers.

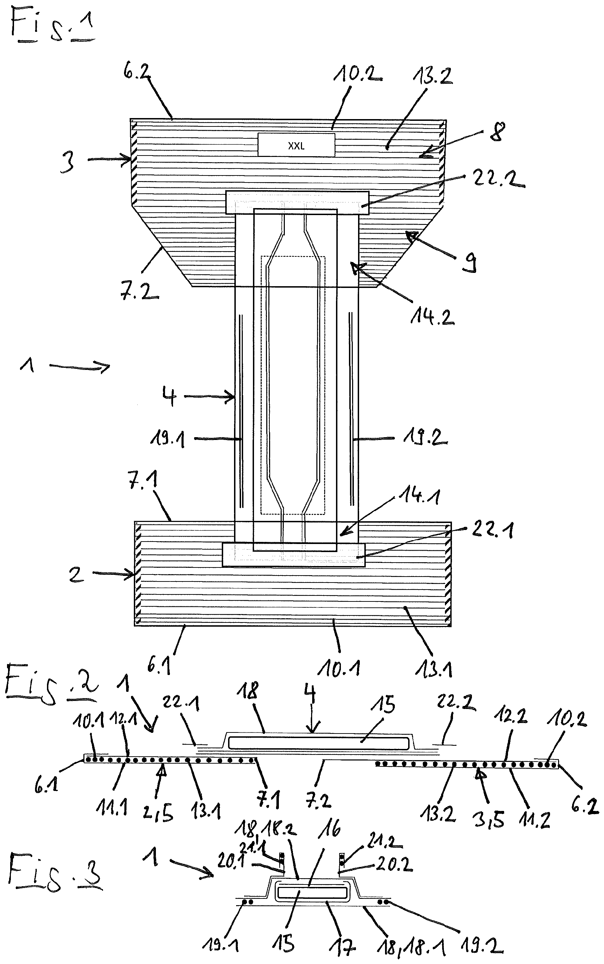

Gemäß Fig. 1 bis 3 umfasst der Hygieneartikel 1 ein Vorderteil 2, ein Rückenteil 3 sowie ein Vorderteil 2 und Rückenteil 3 miteinander verbindendes Einsatzteil 4. Vorderteil 2 und Rückenteil 3 bilden gemeinsam ein Chassis 5.

Das Vorderteil 2 ist rechteckig mit einem oberen Rand 6.1 und einem dazu parallelen unteren Rand 7.1.

Das Rückenteil 3 hat einen rechteckigen oberen Abschnitt 8 mit einem oberen Rand 6.2 und einen im Wesentlichen trapezförmigen unteren Abschnitt 9 mit einem unteren Rand 7.2, der an den Seiten spitzwinklig zum oberen Rand verläuft, wobei die Höhe des Rückenteils 3 zum Einsatzteil 4 hin zunimmt.

Am oberen Rand 6.1, 6.2 haben das Vorderteil 2 und das Rückenteil 3 jeweils einen Bund 10.1, 10.2.

Das Vorderteil 2 und das Rückenteil 3 sind jeweils Laminate, die aus zwei Lagen 11.1, 11.2, 12.1, 12.2 Vliesstoff mit zwischen den beiden Lagen angeordneten elastischen Fäden 13.1, 13.2 bestehen. Die elastischen Fäden 13.1, 13.2 sind parallel zu den oberen Rändern 6.1, 6.2 von Vorderteil 2 und Rückenteil 3 ausgerichtet. Zudem ist der Bund 10.1, 10.2 durch einen gegen die Innenseite umgeschlagenen Randbereich der äußeren Lage des Laminats 11.1, 11.2 begrenzt.

Unterhalb des Bundes 10.1 sind im Vorderteil 2 elastische Fäden 13.1 mit demselben Titer (z. B. 470 dtex) und mit einem konstanten Abstand voneinander (z. B. 4 mm) angeordnet. Die Dichte der elastischen Fäden 13.1 unterhalb des Bundes 10.1 beträgt z. B. 2,5 Fäden pro Zentimeter. Im Bereich des Bundes 10.1 des Vorderteils 2 sind elastische Fäden 13.1 mit demselben Titer und konstantem Abstand voneinander angeordnet. Der Titer dieser Fäden ist größer (z. B. 680 dtex) als der Titer der darunter angeordneten Fäden. Außerdem ist ihr Abstand (z. B. 2,5 mm) geringer als der Abstand der darunter angeordneten Fäden. Zum Beispiel beträgt im Bereich des Bundes 10.1 die Dichte 4 Fäden/cm.

Im Rückenteil 3 sind unterhalb des Bundes 10.2 elastische Fäden 13.2 mit demselben Titer (z. B. 470 dtex) und demselben Abstand (z. B. 4 mm) voneinander angeordnet. Die Dichte der elastischen Fäden 13.2 unterhalb des Bundes 10.2 beträgt z. B. 2,5 Fäden pro Zentimeter. Innerhalb des Bundes 10.2 sind elastische Fäden 13.2 mit demselben Titer und konstantem Abstand voneinander angeordnet. Der Titer dieser elastischen Fäden 13.2 ist größer (z. B. 680 dtex) und der Abstand dieser elastischen Fäden 13.2 ist kleiner (z. B. 2,5 mm) als bei den Fäden unterhalb des Bundes 10.2. Zum Beispiel beträgt im Bereich des Bundes 10.2 die Dichte 4 Fäden/cm.

Die Endbereiche 14.1, 14.2 des Einsatzteils 4 überdecken die unteren Randbereiche von Vorderteil 2 und Rückenteil 3 und sind dort mit diesen mittels Heißleim verklebt. In den Überdeckungsbereichen von Einsatzteil 4 sowie Vorderteil 2 und Rückenteil 3 sind die elastischen Fäden 13.1, 13.2 durchtrennt und nicht an den Laminaten befestigt.

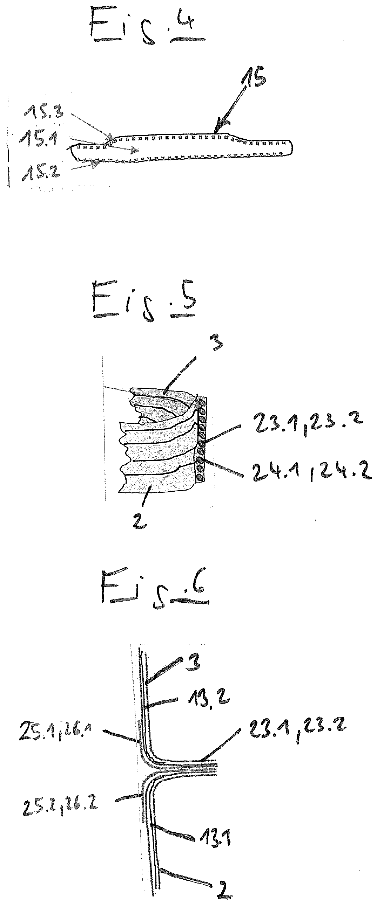

Das Einsatzteil 4 umfasst einen Saugkern 15, der eine mittlere Lage 15.1 aus einem Gemisch aus Cellulosefasern und Superabsorbern und eine untere Lage 15.2 und eine obere Lage 15.3 jeweils aus Cellulosefasern aufweist (vgl. Fig. 4). Der Saugkern 15 hat in der Mitte seine größte Dicke (7 mm) und eine zu den Enden hin sich verringernde Dicke (3-5 mm). Die Dicke des Saugkerns 15 verringert sich zu den Enden hin in den die unteren Randbereiche von Vorderteil 2 und Rückenteil 3 überdeckenden Endbereichen 14.2, 14.2 des Einsatzteils 4.

Auf die Innenseite des Saugkerns 15 ist eine Akquisitionsschicht 16 aufgebracht.

Der Saugkern 15 ist in ein Nonwoven 17 eingeschlagen, dass die Unterseite und die beiden Seitenränder des Saugkerns 15 und die Randbereiche der Akquisitionsschicht 16 überdeckt (C-Einschlag).

Der Saugkern 15, die Akquisitionsschicht 16 und das Nonwoven 17 sind von einen weiteren Nonwoven 18 als Topsheet umschlossen. Das Topsheet hat ein Unterteil 18.1 und ein Oberteil 18.2, die an den beiden Rändern des Saugkerns 15 einander überdecken. Dort sind zwischen Oberteil 18.1 und Unterteil 18.2 in Längsrichtung des Kerns verlaufende elastische Fäden 19.1, 19.2 angeordnet und mittels Heißleim und/oder durch Ultraschallschweißen am weiteren Nonwoven 18 befestigt.

Oberhalb des Saugkerns 15 sind an den beiden Längsseiten des weiteren Nonwovens 18 Abdichtstreifen 20.1, 20.2 aus Nonwoven angeordnet, an deren äußeren Rändern weitere elastische Fäden 21.1, 21.2 befestigt sind, vorzugsweise durch Heißkleben oder durch Ultraschallschweißen.

Die beiden schmalen Ränder des Einsatzteils 4 sind durch streifenförmige Abdeckungen 22.1, 22.2 aus einem Vliesmaterial abgedeckt.

Das Vorderteil 2 und das Rückenteil 3 sind an den beiden vertikalen seitlichen Ränder in Seitennähten 23.1, 23.2 mittels Prägemustern 24.1, 24.2 aneinander befestigt, die durch Heißprägen erzeugt sind (vgl. Fig. 5, 6). In jeder Seitennaht 23.1 23.2 sind zwei Streifen 24.1, 24.2, 25.1, 25.2 aus einem Vliesmaterial eingelegt und durch die Prägemuster 24.1, 24.2 fixiert. Die Streifen 24.1, 24.2, 25.1, 25.2 stehen an den inneren Rändern der Seitennähte 23.1, 23.2 vor und decken die angrenzenden Randbereiche an der Innenseite des Vorderteils 2 und des Rückenteils 3 ab.

Sämtliche elastische Fäden 13, 19, 21 sind im vorgespannten Zustand auf die flachliegenden Materialien aufgebracht, sodass sie diese Materialien bei Entlastung raffen und beim Anlegen gedehnt werden können.

Aufgrund der hohen Dichte der elastischen Fäden 13.1, 13.2 im Bereich von Vorderteil 2 und Rückenteil 3 wird ein hoher Tragekomfort erreicht. Hierzu tragen auch die Ausbildung des Bundes 10 sowie die Abdeckungen 22.1, 22.2 der Enden des Einsatzteils 4 und der Seitennähte 23.1, 23.2 bei.

## Patentansprüche

1. Hygieneartikel in Höschenform mit einem Vorderteil und einem Rückenteil sowie einem Vorderteil und Rückenteil miteinander verbindenden Einsatzteil, wobei Vorderteil und Rückenteil am oberen Rand eine Hüftöffnung umgrenzen, Vorderteil, Rückenteil und Einsatzteil an zwei seitlichen Rändern Beinöffnungen umgrenzen, das Einsatzteil im Schrittbereich einen Saugkern aufweist, Vorderteil und Rückenteil parallel zueinander verlaufende und im entspannten Zustand Vorderteil und Rückenteil raffende elastische Fäden aufweisen und
die elastischen Fäden in senkrecht zum oberen Rand verlaufender Richtung im Mittel eine Dichte von mindestens zwei Fäden pro Zentimeter aufweisen,
**dadurch gekennzeichnet, dass**
das Einsatzteil an den schmalen Seiten durch Streifen aus einem Vliesmaterial abgedeckt ist.

2. Hygieneartikel nach Anspruch 1, bei dem die elastischen Fäden entlang von zum oberen Rand von Vorderteil und Rückenteil parallelen Achsen verlaufen.

3. Hygieneartikel nach Anspruch 2, bei dem die elastischen Fäden geradlinig oder wellenförmig entlang der Achsen verlaufen.

4. Hygieneartikel nach einem der Ansprüche 1 bis 3, bei dem die elastischen Fäden in einer senkrecht zum oberen Rand verlaufenden Richtung zumindest in einem Abschnitt des Vorderteils und des Rückenteils gleichmäßig über das Vorderteil und das Rückenteil verteilt sind.

5. Hygieneartikel nach einem der Ansprüche 1 bis 4, bei dem die elastischen Fäden in einer senkrecht zum oberen Rand verlaufenden Richtung zumindest über einen Abschnitt des Vorderteils und des Rückenteils denselben Titer aufweisen.

6. Hygieneartikel nach einem der Ansprüche 1 bis 5, bei dem das Vorderteil und/oder das Rückenteil ein Laminat aus zwei Lagen Vliesstoff mit zwischen den beiden Lagen angeordneten und an mindestens einer Lage befestigten elastischen Fäden umfassen oder durch ein solches Laminat gebildet sind.

7. Hygieneartikel nach Anspruch 6, bei dem die elastischen Fäden an mindestens einer Lage aus Vliesstoff angeklebt und/oder durch Ultraschallschweißen an mindestens einer Lage aus Vliesstoff befestigt sind.

8. Hygieneartikel nach einem der Ansprüche 1 bis 7, bei dem die elastischen Fäden im gedehnten Zustand auf eine Lage aus einem flach ausgebreiteten Vliesstoff aufgebracht und an dieser befestigt sind.

9. Hygieneartikel nach Anspruch 8, bei dem die elastischen Fäden in einer senkrecht zum oberen Rand verlaufenden Richtung überall mit derselben Vorspannung auf eine Lage aus einem Vliesstoff aufgebracht und an dieser befestigt sind.

10. Hygieneartikel nach einem der Ansprüche 1 bis 9, bei dem das Vorderteil und das Rückenteil am oberen Rand einen elastischen Bund aufweisen.

11. Hygieneartikel nach Anspruch 10, bei dem das Vorderteil und das Rückenteil innerhalb des elastischen Bundes parallel zueinander verlaufende und im entspannten Zustand den Bund raffende elastische Fäden aufweisen.

12. Hygieneartikel nach einem der Ansprüche 1 bis 11, bei dem das Vorderteil rechteckig ist und/oder bei dem das Rückenteil in einem oberen Abschnitt rechteckig und in einem unteren Abschnitt trapezförmig ist.

13. Hygieneartikel nach einem der Ansprüche 1 bis 12, bei dem das Vorderteil und das Rückenteil in Seitennähten an den seitlichen Rändern durch Heißkleber und/oder Prägemuster miteinander verbunden sind, wobei vorzugsweise in den Seitennähten zwischen den einander überlappenden seitlichen Rändern von Vorderteil und Rückenteil Streifen aus einem Vliesmaterial eingelegt sind, die von den inneren Rändern der Seitennähte vorstehen und die angrenzenden Randbereiche des Vorderteils und des Rückenteils überdecken.

14. Hygieneartikel nach einem der Ansprüche 1 bis 13, bei dem Endbereiche des Einsatzteils untere Randbereiche des Vorderteils und des Rückenteiles überdecken, an diesen befestigt sind und die die elastischen Fäden im Bereich des Einsatzteils durchtrennt sind.

15. Hygieneartikel nach Anspruch 14, bei dem die elastischen Fäden im Bereich des Einsatzteils nicht an den Lagen aus Vliesstoff befestigt sind.

16. Hygieneartikel nach einem der Ansprüche 1 bis 15, bei dem die elastischen Fäden Elasthanfäden oder Gummifäden sind.

17. Hygieneartikel nach einem der Ansprüche 1 bis 16 umfassend mindestens eines der folgenden Merkmale:
- die elastischen Fäden weisen in senkrecht zum oberen Rand verlaufender Richtung im Mittel eine Dichte von mindestens 2,5 Fäden pro Zentimeter, vorzugsweise mindestens vier Fäden pro Zentimeter, vorzugsweise mindestens fünf Fäden pro Zentimeter, vorzugsweise mindestens acht Fäden pro Zentimeter, vorzugsweise mindestens zehn Fäden pro Zentimeter auf,
- der Saugkern umfasst mindestens eine Lage aus einem Gemisch aus Cellulosefasern und Superabsorbern,
- der Saugkern hat in der Mitte seine größte Dicke und eine zu den Enden hin sich verringernde Dicke.
- die Dicke des Saugkerns verringert sich zu den Enden des Saugkerns hin in den die unteren Randbereiche von Vorderteil und Rückenteil überdeckenden Endbereichen des Einsatzteils,
- der Saugkern ist in ein Nonwoven eingeschlagen, wobei das Nonwoven den Saugkern vorzugsweise C-förmig umhüllt,
- auf die Innenseite des Saugkerns ist eine Akquisitionsschicht aufgebracht, wobei die Akquisitionsschicht vorzugsweise auf der offenen Seite eines den Saugkern C-förmig umhüllenden Nonwovens angeordnet ist,
- der Saugkern und die Akquisitionsschicht sind von einem weiteren Nonwoven als Topsheet umschlossen,
- an den seitlichen Randbereichen des Einsatzteils sind elastische Fäden aufgebracht.
- die elastischen Fäden sind zwischen zwei Lagen des weiteren Nonwovens angeordnet und an mindestens einer dieser Lagen befestigt,
- auf der Innenseite des Einsatzteils sind den beiden Längsseiten des Saugkerns Abdichtstreifen (Cuffs) angeordnet, an deren äußeren Rändern elastische Fäden befestigt sind.

## Claims

1. A hygiene article in panty form, having a front part and a rear part as well as an insert part which connects the front part and the rear part to one another, wherein the front part and the rear part delimit a hip opening at the upper edge, the front part, the rear part and the insert part delimit leg openings at two lateral edges, the insert part has an absorbent core in the crotch region, the front part and the rear part have elastic threads which run parallel to one another and which, in the relaxed state, gather the front part and the rear part, and the elastic threads have, on average, a density of at least two threads per centimeter in a direction running perpendicularly to the upper edge, **characterized in that** the insert part is covered by strips made of a nonwoven material on the narrow sides.

2. The hygiene article according to Claim 1, in which the elastic threads run along axes parallel to the upper edge of the front part and the rear part.

3. The hygiene article according to Claim 2, in which the elastic threads run in a straight line or in an undulating manner along the axes.

4. The hygiene article according to any one of Claims 1 to 3, in which the elastic threads are uniformly distributed over the front part and the rear part in a direction running perpendicularly to the upper edge at least in a section of the front part and of the rear part.

5. The hygiene article according to any one of Claims 1 to 4, in which the elastic threads have the same titer in a direction running perpendicularly to the upper edge, at least over a section of the front part and of the rear part.

6. The hygiene article according to any one of Claims 1 to 5, in which the front part and/or the rear part comprise a laminate made of two layers of nonwoven fabric with elastic threads arranged between the two layers and fastened to at least one layer or are formed by such a laminate.

7. The hygiene article according to Claim 6, in which the elastic threads are glued to at least one layer made of nonwoven fabric and/or are fastened to at least one layer made of nonwoven fabric by ultrasonic welding.

8. The hygiene article according to any one of Claims 1 to 7, in which the elastic threads, in the stretched state, are applied to a layer made of a nonwoven fabric spread out flat and are fastened thereto.

9. The hygiene article according to Claim 8, in which the elastic threads are applied to a layer made of a nonwoven fabric and are fastened thereto in a direction running perpendicularly to the upper edge with the same pretension everywhere.

10. The hygiene article according to any one of Claims 1 to 9, in which the front part and the rear part have an elastic waistband at the upper edge.

11. The hygiene article according to Claim 10, in which the front part and the rear part have elastic threads which run parallel to one another within the elastic waistband and which, in the relaxed state, gather the waistband.

12. The hygiene article according to any one of Claims 1 to 11, in which the front part is rectangular and/or in which the rear part is rectangular in an upper section and trapezoidal in a lower section.

13. The hygiene article according to any one of Claims 1 to 12, in which the front part and the rear part are connected to one another in side seams on the lateral edges by hot glue and/or embossed patterns, wherein strips made of a nonwoven material are preferably inserted in the side seams between the overlapping lateral edges of the front part and of the rear part, which strips protrude from the inner edges of the side seams and cover the adjacent edge regions of the front part and of the back part.

14. The hygiene article according to any one of Claims 1 to 13, in which end regions of the insert part cover lower edge regions of the front part and of the rear part, are fastened to these and the elastic threads are severed in the region of the insert part.

15. The hygiene article according to Claim 14, in which the elastic threads are not fastened to the layers made of nonwoven fabric in the region of the insert part

16. The hygiene article according to any one of Claims 1 to 15, in which the elastic threads are spandex threads or rubber threads.

17. The hygiene article according to any one of Claims 1 to 16, comprising at least one of the following features:
- the elastic threads have, on average, a density of at least 2.5 threads per centimeter, preferably at least four threads per centimeter, preferably at least five threads per centimeter, preferably at least eight threads per centimeter, preferably at least ten threads per centimeter in a direction running perpendicularly to the upper edge,
- the absorbent core comprises at least one layer made of a mixture of cellulose fibers and super absorbers,
- the absorbent core has its greatest thickness in the center and a thickness which decreases towards the ends,
- the thickness of the absorbent core decreases towards the ends of the absorbent core in the end regions of the insert, which cover the lower edge regions of the front part and of the back part,
- the absorbent core is enveloped by a nonwoven, wherein the nonwoven preferably encases the absorbent core in a C-shape,
- an acquisition layer is applied to the inside of the absorbent core, wherein the acquisition layer is preferably arranged on the open side of a nonwoven encasing the absorbent core in a C-shape,
- the absorbent core and the acquisition layer are surrounded by a further nonwoven as a top sheet,
- elastic threads are applied to the lateral edge regions of the insert part,
- the elastic threads are arranged between two layers of the further nonwoven and fastened to at least one of these layers,
- sealing strips (cuffs) are arranged on the inside of the insert part, on the two longitudinal sides of the absorbent core, elastic threads being fastened to the outer edges of said sealing strips.

## Revendications

1. Article d'hygiène sous forme de culotte comprenant une partie avant et une partie arrière ainsi qu'une partie d'insert reliant la partie avant et la partie arrière, dans lequel la partie avant et la partie arrière délimitent une ouverture de hanche sur le bord supérieur, la partie avant, la partie arrière et la partie d'insert délimitent une ouverture de jambe sur deux bords latéraux, la partie d'insert présente un noyau absorbant dans la zone d'entrejambe, la partie avant et la partie arrière présentent des fils élastiques s'étendant parallèlement les uns aux autres et fronçant la partie avant et la partie arrière dans l'état détendu et
les fils élastiques présentent en moyenne une densité d'au moins deux fils par centimètre dans la direction perpendiculaire au bord supérieur,
**caractérisé en ce que**
la partie d'insert est recouverte par des bandes de matière non tissée sur les côtés étroits.

2. Article d'hygiène selon la revendication 1, dans lequel les fils élastiques s'étendent le long d'axes parallèles au bord supérieur de la partie avant et de la partie arrière.

3. Article d'hygiène selon la revendication 2, dans lequel les fils élastiques s'étendent de façon rectiligne ou en forme d'onde le long des axes.

4. Article d'hygiène selon l'une des revendications 1 à 3, dans lequel les fils élastiques sont répartis uniformément sur la partie avant et la partie arrière dans une direction perpendiculaire au bord supérieur au moins dans une section de la partie avant et de la partie arrière.

5. Article d'hygiène selon l'une des revendications 1 à 4, dans lequel les fils élastiques présentent la même densité linéaire dans une direction perpendiculaire au bord supérieur au moins sur une section de la partie avant et de la partie arrière.

6. Article d'hygiène selon l'une des revendications 1 à 5, dans lequel la partie avant et/ou la partie arrière comportent un stratifié constitué de deux couches de non tissé avec des fils élastiques disposés entre les deux couches et fixés à au moins une couche et sont formées par un tel stratifié.

7. Article d'hygiène selon la revendication 6, dans lequel les fils élastiques sont collés à au moins une couche de non tissé et/ou fixés à au moins une couche de non tissé par soudage aux ultrasons.

8. Article d'hygiène selon l'une des revendications 1 à 7, dans lequel les fils élastiques sont appliqués dans l'état détendu sur une couche constituée d'un non tissé étendu à plat et fixés à celle-ci.

9. Article d'hygiène selon la revendication 8, dans lequel les fils élastiques sont appliqués sur une couche de non tissé partout avec la même précontrainte dans une direction perpendiculaire au bord supérieur et fixés à celle-ci.

10. Article d'hygiène selon l'une des revendications 1 à 9, dans lequel la partie avant et la partie arrière présentent une ceinture élastique sur le bord supérieur.

11. Article d'hygiène selon la revendication 10, dans lequel la partie avant et la partie arrière présentent des fils élastiques s'étendant parallèlement les uns aux autres dans la ceinture élastique et fronçant la ceinture dans l'état détendu.

12. Article d'hygiène selon l'une des revendications 1 à 11, dans lequel la partie avant est rectangulaire et/ou dans lequel la partie arrière est rectangulaire dans une section supérieure et trapézoïdale dans une section inférieure.

13. Article d'hygiène selon l'une des revendications 1 à 12, dans lequel la partie avant et la partie arrière sont reliées entre elles dans des coutures latérales sur les bords latéraux par un adhésif thermofusible et/ou un motif gaufré, dans lequel des bandes constituées d'un non tissé, faisant saillie à partir des bords intérieurs des coutures latérales et recouvrant les zones de bord adjacentes de la partie avant et de la partie arrière, sont insérées avantageusement dans les coutures latérales entre les bords latéraux superposés entre eux de la partie avant et de la partie arrière.

14. Article d'hygiène selon l'une des revendications 1 à 13, dans lequel des zones d'extrémité de la partie d'insert recouvrent des zones de bord inférieures de la partie avant et de la partie arrière, sont fixées à celles-ci et les fils élastiques sont sectionnés dans la zone de la partie d'insert.

15. Article d'hygiène selon la revendication 14, dans lequel les fils élastiques ne sont pas fixés aux couches de non tissé dans la zone de la partie d'insert.

16. Article d'hygiène selon l'une des revendications 1 à 15, dans lequel les fils élastiques sont des fils d'élasthanne ou des fils de caoutchouc.

17. Article d'hygiène selon l'une des revendications 1 à 16, comportant l'une au moins des caractéristiques suivantes :
- les fils élastiques présentent en moyenne une densité d'au moins 2,5 fils par centimètre dans la direction perpendiculaire au bord supérieur, avantageusement d'au moins quatre fils par centimètre, avantageusement d'au moins cinq fils par centimètre, avantageusement d'au moins huit fils par centimètre, avantageusement d'au moins dix fils par centimètre,
- le noyau absorbant comporte au moins une couche constituée d'un mélange de fibres de cellulose et de super-absorbants,
- le noyau absorbant présente son épaisseur maximale en son centre et une épaisseur décroissante vers les extrémités,
- l'épaisseur du noyau absorbant décroit vers les extrémités du noyau absorbant dans les zones d'extrémité de la partie d'insert recouvrant les zones de bord inférieures de la partie avant et de la partie arrière,
- le noyau absorbant est enfoncé dans un non tissé, le non tissé enveloppant le noyau absorbant avantageusement en forme de C,
- une couche d'acquisition est appliquée sur le côté intérieur du noyau absorbant, la couche d'acquisition étant avantageusement disposée sur le côté ouvert d'un non tissé enveloppant le noyau absorbant en forme de C,
- le noyau absorbant et la couche d'acquisition sont entourés par un non tissé supplémentaire en tant que couche supérieure,
- des fils élastiques sont appliqués sur les zones de bord latérales de la partie d'insert,
- les fils élastiques sont disposés entre deux couches du non tissé supplémentaire et fixés à l'une au moins de ces couches,
- sur le côté intérieur de la partie d'insert, des bandes d'étanchéité (manchons) sur les bords extérieurs desquelles sont fixés des fils élastiques sont disposées sur les deux côtés longitudinaux du noyau absorbant.
